# EUROPEAN PATENT APPLICATION

(11) **EP 4 691 540 A1**
(43) Date of publication of application: **11.02.2026**
(21) Application number: 24199022.5
(22) Date of filing: 06.09.2024
(51) Int. Cl.: A61M 60/104, A61M 60/13, A61M 60/274, A61M 60/31, A61M 60/427, A61M 60/531, A61M 60/837

(54) **OUTSIDE AORTIC COUNTERPULSATION DEVICE WITH DIAPHRAGM CAVITY**

(30) Priority: 05.08.2024 CN 202411065503
(71) Applicant: Taizhou MYDsn Medical Technology Co., Ltd, Taizhou Jiangsu 225316 (CN)
(72) Inventor: TAN, Xuerui, Beijing, 100043 (CN); JIA, Haitao, Tianjin, 301700 (CN); HE, Guojun, Wuhan, 430000 (CN)
(74) Representative: Murgitroyd & Company

(57) **Abstract**

The present invention discloses an outside aortic counterpulsation device with a diaphragm cavity, including an in vivo catheter, the diaphragm cavity, an in vitro catheter and a controller that are connected in sequence, wherein a side wall of an extending end of the in vivo catheter is provided with a plurality of through holes; the diaphragm cavity is divided by a first diaphragm into a blood cavity and an inflation cavity, the blood cavity is communicated to the in vivo catheter, and the inflation cavity is connected to the controller through the in vitro catheter. The controller supplies an inflation or deflation gas to the inflation cavity; during inflation of the inflation cavity, a space of the blood cavity is occupied to push blood in the blood cavity back into a body, and during deflation of the inflation cavity, the space of the blood cavity increases to draw blood from a descending aorta into the blood cavity, achieving an outside aortic blood counterpulsation effect. According to the present invention, the inflation cavity is placed in vitro by means of the diaphragm cavity to achieve outside aortic blood counterpulsation, and the volume of the diaphragm cavity and the volume of the inflation cavity are reasonably arranged based on the principle that the in vitro diaphragm cavity is not limited by an in vivo space, such that the cardiac output is greatly improved to meet the patient need for systemic blood supply.

## Description

### BACKGROUND OF THE INVENTION

### 1. Technical Field

The present invention relates to the technical field of medical devices, and in particular to an outside aortic counterpulsation device with a diaphragm cavity.

### 2. Description of Related Art

Intra-aortic balloon pump, a mechanical device designed according to the principle of counterpulsation to assist the failing left heart, is typically used for in-hospital rehabilitation after interventional heart surgery, heart attack or other major cardiac events. It is the most commonly used auxiliary device in the perioperative period of PCI, particularly suitable for providing advanced life support for critically ill cardiac patients, and can effectively improve the coronary blood supply as well as low cardiac output and hypotension for the patients. The basic principle of IABP is as follows: a balloon is disposed in the descending aorta to inflate during diastole and deflate during systole, achieving the effect of assisting cardiac circulation. Inflating the intra-aortic balloon during diastole can increase the diastolic blood pressure and thus the blood flow in the coronary artery to improve myocardial blood and oxygen supply while increasing the blood perfusion to the brain, kidneys and peripheries; and quickly retracting and emptying the balloon during systole can induce "cavitation" to reduce the left ventricular afterload and thus reduce myocardial oxygen consumption.

In the interventional therapies for cardiovascular diseases, IABP is the most commonly used auxiliary device in the perioperative period of PCI. According to the data from the National Cardiovascular Data Registry of the United States from 2009 to 2013, 89.3% of patients supported by pMCS used IABPs, and only 10.7% used other auxiliary devices. At present, all the IABPs in China rely on imports. Major manufacturers across the world involves three brands, including Getinge Group, Teleflex, and Senko Medical Instrument Mfg. Co., Ltd (MERA). In 2020, the share of Getinge accounted for more than 80%. As for the selection criteria for IABPs, the size of a balloon is determined depending on height. At present, Teleflex's Arrow IABP system has the catheter available with the diameter of 7 fr, 7.5 fr and 8fr, and the balloon available with the capacity of 30 cc, 40 cc and 50cc.

From the comparison of IABP, ECMO, and artificial heart in usage, IABP shows absolute advantages in sheath size, bedside placement, operation difficulty, indwelling time, postoperative management requirements, hemolysis risk or the like, with the sole defect of excessively low cardiac output, which is 0.5-1 L per minute. Consequently, it is significantly inferior to ECMO and the artificial heart in the actual use effect on the patients. In view of this major defect of the IABP device, international and national physicians and engineers have tried many methods, but with little effect. As a result, there is no effective method to solve the problem of excessively low cardiac output of the IABP device.

On this basis, the present application makes full use of the advantages of the IABP device to creatively propose an outside aortic counterpulsation device with a diaphragm cavity, in which an inflation cavity is disposed in a diaphragm cavity in vitro in an outside aortic counterpulsation mode to maximize the cardiac output and solve the problem of excessively low cardiac output of an IABP device.

### BRIEF SUMMARY OF THE INVENTION

The technical problem to be solved by the present invention is to provide an outside aortic counterpulsation device with a diaphragm cavity, in which an inflation cavity disposed in an in vitro diaphragm cavity in an outside aortic counterpulsation mode is enabled to maximize the cardiac output and solve the problem of excessively low cardiac output of an IABP device.

To solve the technical problem above, the present invention provides an outside aortic counterpulsation device with a diaphragm cavity, including an in vivo catheter, the diaphragm cavity, an in vitro catheter and a controller that are connected in sequence,
wherein the in vivo catheter is used for extending into a descending aorta, and a side wall of an extending end of the in vivo catheter is provided with a plurality of through holes;
the diaphragm cavity includes a cavity body and a first diaphragm disposed inside the cavity body, the first diaphragm is an elastic diaphragm body for dividing the cavity body into a blood cavity and an inflation cavity, the blood cavity is in airtight connection with an in vitro end of the in vivo catheter by means of a first fitting of the cavity body, the inflation cavity is in airtight connection with an end of the in vitro catheter by means of a second fitting of the cavity body, and the other end of the in vitro catheter is connected to the controller; and
the controller supplies an inflation or deflation gas to the inflation cavity, during inflation of the inflation cavity, the first diaphragm bulges towards the blood cavity, a space of the blood cavity is occupied to push blood in the blood cavity back into a body, and during deflation of the inflation cavity, the first diaphragm retracts, and the space of the blood cavity increases to draw blood from the descending aorta into the blood cavity, achieving an outside aortic blood counterpulsation effect.

As a further improvement, the first diaphragm is made of silicone rubber, polyurethane, PTFE or other elastic materials having a hardness of less than 30, with a thickness of 0.3-2 mm, and a gas volume of the inflation cavity is more than 150 ml after inflation.

As a further improvement, the cavity body is made of a rigid PEEK, PPSU, PU, PC or PE material, or a silicone rubber material having a hardness of more than 65.

As a further improvement, the first fitting and the second fitting of the cavity body are each a Luer fitting.

As a further improvement, a pressure sensor is disposed on an outer side of a tip of an inner end of the in vivo catheter.

As a further improvement, the in vivo catheter has a lumen diameter of 2.2-9 mm, a perforated section of the in vivo catheter has a length of 320-520 mm, each of the through holes has a diameter of 1-4 mm, and a spacing between every two adjacent through holes is 1-10 mm.

As a further improvement, a second diaphragm for dividing an interior of the in vivo catheter into a blood drawing cavity and a perfusion cavity is further disposed inside the in vivo catheter, one end of the second diaphragm is located at a beginning of the perforated section of the in vivo catheter, and the other end of the second diaphragm is located at a tip of the in vitro end of the in vivo catheter.

As a further improvement, both sides of the second diaphragm are adhered to an inner side wall of the in vivo catheter respectively, and both ends of the second diaphragm are inclined such that an inner end of the blood drawing cavity and an outer end of the perfusion cavity are each of a flared structure.

As a further improvement, the second diaphragm is made of an elastic thin film.

As a further improvement, the in vivo catheter and the in vitro catheter are each made of a polymer material such as PTFE, PU, Pebax or PE and prepared by a blow molding or extrusion process, and the second diaphragm is made of silicone rubber, polyurethane, PTFE, PU, Pebax, PE, or other elastic medical polymer materials having a hardness of less than 30. In such a design, the present invention at least has the following advantages.
1. The outside aortic counterpulsation device with the diaphragm cavity according to the present invention can allow for the flow of blood from the descending aorta between the in vivo catheter and the blood cavity of the diaphragm cavity by means of the in vitro diaphragm cavity, achieving the in vitro counterpulsation of the outside aortic blood. According to the outside aortic counterpulsation device with the diaphragm cavity, the first diaphragm in the diaphragm cavity is disposed such that the volume of the inflation cavity in the diaphragm cavity can greatly increase to significantly improve the cardiac output of the outside aortic counterpulsation, thus truly solving the problem of low cardiac output caused by limited volume of the existing built-in balloon and also solving the problem of limitation of small change of the cavity body of the existing diaphragm cavity. That is, the inflation volume of the improved diaphragm cavity is easier to control, which allows the cardiac output to be more than 5 L/min, optimally up to 9 L/min, meeting the patient need for systemic blood supply and greatly improving the blood supply condition of the patient. In addition, the device allows the inflation cavity to keep away from the aortic blood vessels, such that blockage can be performed in time even in the case of a high-risk event such as the gas leakage of the inflation cavity, thereby preventing adverse effects on the patient to achieve high safety.
2. Furthermore, the diaphragm is disposed in the in vivo catheter to form a dual-channel structure in the in vivo catheter, allowing for blood drawing at one side and blood perfusion at the other side, such that the two channels do not affect each other to avoid problems such as blood collision and serious turbulence in the case of a single channel.
3. Furthermore, the elastic thin film is disposed with both ends provided with flared structures, which can effectively guarantee the unidirectional flow of blood and allow for maximum unidirectional cross section of the blood during drawing and perfusion, thereby greatly reducing the external power for better implementation of the unidirectional flow of blood.

### BRIEF DESCRIPTION OF THE SEVERAL VIEWS OF THE DRAWINGS

Described above is only an outline of the technical solution of the present invention. For clearer understanding of the technical means of the present invention, the present invention will be further illustrated in detail below in conjunction with the accompanying drawings and the specific embodiments.
Fig. 1 is a schematic structural diagram of an outside aortic counterpulsation device with a diaphragm cavity, applied to a human body, according to the present invention;
Fig. 2 is a schematic structural diagram of an in vivo catheter in the outside aortic counterpulsation device with the diaphragm cavity according to the present invention;
Fig. 3 is a schematic structural diagram of the diaphragm cavity, with an inflation cavity inflated, in the outside aortic counterpulsation device with the diaphragm cavity according to the present invention;
Fig. 4 is a schematic structural diagram of the diaphragm cavity, with the inflation cavity deflated, in the outside aortic counterpulsation device with the diaphragm cavity according to the present invention;
Fig. 5 is a schematic structural diagram of a diaphragm section of the in vivo catheter in the outside aortic counterpulsation device according to the present invention;
Fig. 6 is a schematic section view of the diaphragm section of the in vivo catheter in the outside aortic counterpulsation device according to the present invention (with a blood drawing cavity expanded); and
Fig. 7 is a schematic section view of the diaphragm section of the in vivo catheter in the outside aortic counterpulsation device according to the present invention (with a perfusion cavity expanded).

### DETAILED DESCRIPTION OF THE INVENTION

The exemplary embodiments of the present invention will be described below in more details with reference to the accompanying drawings. Although the exemplary embodiments of the present invention are shown in the accompanying drawings, it should be understood that the present invention can be implemented in various forms and should not be limited by the embodiments set forth herein. On the contrary, these embodiments are provided to enable a more thorough understanding of the present invention and to fully convey the scope of the present invention to those skilled in the art.

It should also be noted that the terms such as "left", "right", "inside", and "outside" in the present application indicate directions or positional relations based on the directions or positional relations as shown in the accompanying drawings only for the convenience of describing the present invention and simplifying the description, instead of indicating or implying that a mentioned device or element must have a specific direction or must be constructed and operated in a specific direction. Therefore, these terms should not be construed as limiting the present invention.

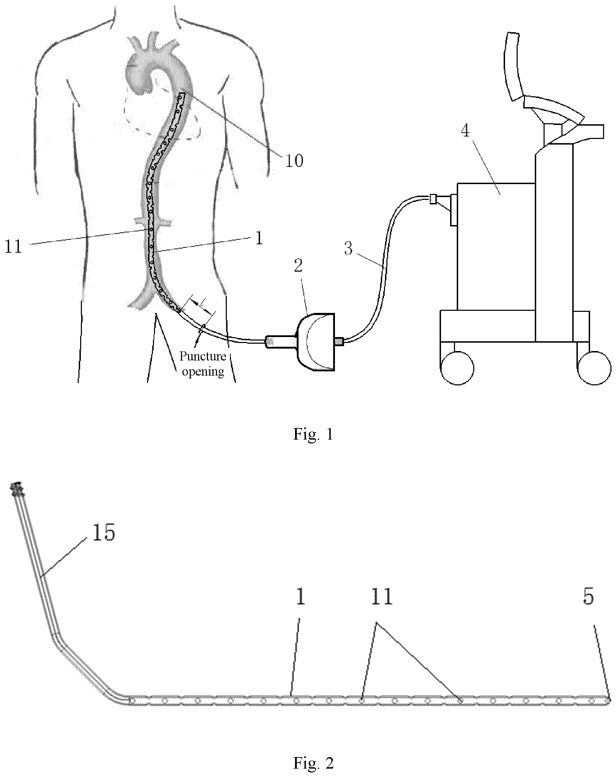

Referring to Fig. 1, an outside aortic counterpulsation device with a diaphragm cavity in this embodiment includes an in vivo catheter 1, the diaphragm cavity 2, an in vitro catheter 3 and a controller 4 that are connected in sequence.

Referring to Fig. 2, the in vivo catheter 1 is used for extending into a descending aorta 10, the side wall of an extending end of the in vivo catheter 1 is provided with several through holes 11, and an in vitro end of the in vivo catheter is provided as a seal fitting, preferably a Luer fitting, for airtight connection with the diaphragm cavity 2.

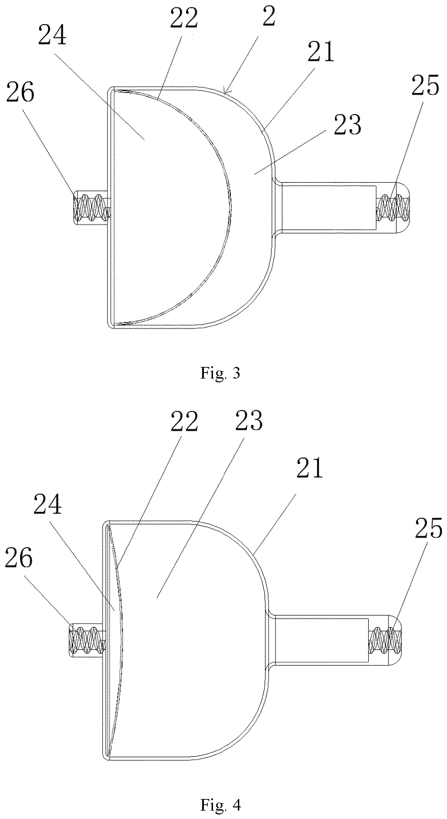

Referring to Figs. 3 and 4, the diaphragm cavity 2 includes a cavity body 21 and a first diaphragm 22 disposed inside the cavity body 21. The first diaphragm 22 is an elastic diaphragm body dividing the cavity body 21 into a blood cavity 23 and an inflation cavity 24. The blood cavity 23 is in airtight connection with the in vitro end of the in vivo catheter 1 by means of a first fitting 25 of the cavity body 21, the inflation cavity 24 is in airtight connection with an end of the in vitro catheter 3 by means of a second fitting 26 of the cavity body, and the other end of the in vitro catheter 3 is connected to the controller 4. The first and second fittings 25 and 26 of the cavity body 21 are each preferably a Luer fitting to ensure both strength and airtightness of the connection.

The controller 4 supplies an inflation or deflation gas to the inflation cavity 24; during inflation of the inflation cavity 24, the first diaphragm 22 bulges towards the blood cavity 22, a space of the blood cavity 23 is occupied to push blood in the blood cavity 23 back into a body, which is a perfusion state; and during deflation of the inflation cavity 24, the first diaphragm 22 retracts, and the space of the blood cavity 23 increases to draw blood from the descending aorta 10 into the blood cavity 23 by means of "cavitation", which is a drawing state, thereby achieving an outside aortic blood counterpulsation effect. That is, the inflation cavity 24 is used for accommodating a gas delivered from the controller 4, and has two states, namely, inflation and deflation. The blood cavity 23 is used for receiving and accommodating blood introduced from the aortic blood vessels of a human body, and has two states, namely, drawing and perfusion. The inflation cavity 24 and the blood cavity 23 are completely isolated by means of a diaphragm 22 therebetween, without any chance of contact, such that the blood cavity 23 and the in vivo catheter 1 form a closed cavity to achieve a better effect than that of a sealing means such as a hemostatic valve.

That is, when the in vivo catheter 1 enters the body, the in vivo catheter 1 and the blood cavity 23, together with the blood vessels in the body, form a closed space, and blood flows in pathways formed in the closed space. This space is an extension section of the aortic blood vessel of the human body. Of course, the diaphragm cavity 2 may be connected to the in vivo catheter 1 and the in vitro catheter 3 by means of any other existing sealing and connecting mechanisms, for example, threaded connection, as long as the strength and airtightness of the connection are guaranteed.

In this embodiment, the first diaphragm 22 is made of silicone rubber, polyurethane, PTFE or other elastic materials having a hardness of less than 30, with a thickness of 0.3-2 mm, preferably 0.5 mm, thereby ensuring that the energy demand is low and the diaphragm is not easy to break, during inflation. The cavity body 21 is made of PEEK, PPSU, PU, PC, PE or other materials having certain rigidity, or a silicone rubber material having certain elasticity. The cavity body 21 is made of a material having certain elasticity, mainly because it can resist the impact force produced during blood drawing or perfusion to achieve a buffering effect. With the buffering effect, the cavity can protect the structure of the overall diaphragm cavity 2, but may reduce the perfusion efficiency and also lead to increased energy demand. Therefore, it is more appropriate to choose the silicone rubber material with the hardness of more than 65.

Specifically, the first diaphragm 22 may be integrally molded with the cavity body 21, or may be molded individually. During individual molding, the periphery of the first diaphragm 22 is adhered to the inner wall of the cavity body 21, as long as the first diaphragm can divide the inner cavity of the cavity body.

According to the outside aortic counterpulsation device with the diaphragm cavity, since the inflation cavity 24 is disposed in vitro, the size, shape and volume of the diaphragm cavity 2 can be configured freely, making it possible to acquire the maximum cardiac output. As in this embodiment, the diaphragm cavity 2 is provided as a cavity with a volume of greater than 200 ml, the inflation cavity 24 in an inflation state has a volume of more than 150 ml, with the maximum volume up to 200 ml, such that the space of the blood cavity 23 can be completely filled, and the blood can be completely pushed back to the body from the diaphragm cavity 2, avoiding the thrombosis caused by blood retention in the diaphragm cavity. Moreover, the diaphragm cavity 2 in this embodiment may be shaped as a cylinder, a sphere, a hemisphere, a round table, an oval, or the like.

More specifically, the overall length of the in vivo catheter 1 is 500-1000 mm, preferably 600 mm, 750 mm or 900 mm, so as to adapt to the human body of a different height. The outer diameter of the in vivo catheter 1 is at least 7 F, and at most 24 F. That is, the in vivo catheter has a lumen diameter of 2.2-9 mm. The principle for setting the diameter of the in vivo catheter is that: the inner lumen diameter of the aortic blood vessel in a human body is generally about 10 mm, the outer diameter of the catheter must be smaller than the inner diameter of the aortic blood vessel, with a certain gap reserved, and a gap of 0.5 mm at one side is necessary, therefore, the outer diameter of the catheter cannot exceed 9 mm at most, otherwise the inner wall of the aortic blood vessel will be damaged. Of course, the catheter should not be too thin, and if so, it is laborious to draw and perfuse the blood and the load is very large; moreover, liquid hysteresis becomes very significant; and meanwhile, vacuum may occur during vigorous drawing, which will damage blood cells or induce aeroembolism to endanger the life safety of patients.

Also, a perforated section of the in vivo catheter 1 provided with the through holes 11 has a length of 320-520 mm. A plurality of the through holes 11 are provided for reducing the load during blood drawing and perfusion, allowing for the flow of blood easily. The through holes 11 each have a diameter of 1-4 mm and are evenly distributed around the wall of the in vivo catheter in a mode that is not limited. The through holes may be distributed spirally, equidistantly in circles, or optimally in such a way to ensure an equal distance between any two through holes, with the distance in a range of 1-10 mm. In case of a too small distance, the overall rigidity of the catheter may be destroyed, and in case of a too large distance, the drawing load may be increased. The same is true for the aperture, where in case of a too small aperture, the drawing load increases, leading to increased difficulty, and in case of a too large aperture, the overall rigidity will be destroyed, making the catheter easy to damage. Notably, the initial position for perforation in the wall of the catheter should not be too close to a puncture opening for accessing to the body, otherwise, oozing of blood may be caused easily. The optimal range of L from an initial perforation section to the position of a puncture point is: 20-80 mm. In case of a too short distance, oozing of blood may be caused due to high pressure during blood drawing and perfusion, and in case of a too long distance, a blind cavity region unperforated will become large, leading to increased drawing load and thus increased difficulty in drawing. The optimal range of L is 30-50 mm.

In this embodiment, a pressure sensor 5 is disposed on the outer side of a tip of an inner end of the in vivo catheter 1 for monitoring the blood pressure condition in real time and transmitting signals to the controller 4 to create a control trigger mode.

The application principle of the outside aortic counterpulsation device with the diaphragm cavity is that: the in vivo catheter 1 is disposed in the aorta through the puncture surgery, and the end of the in vivo catheter is located in the descending aorta 10 and reaches at most, without entering, the aortic arch, preferably at a distance of about 100 mm from the bend of the aortic arch. The surgical puncture may be performed in the femoral artery, the radial artery, or the carotid artery. After the puncture is completed for the in vivo catheter 1, the blood in vivo is directed along the catheter into the blood cavity 23 of the diaphragm cavity 2. The inflation and deflation of the inflation cavity 24 are controlled by means of the controller 4 to direct the blood from the descending aorta 10 in vivo to the in vitro blood cavity 23 along the catheter via the through holes 11, and then perfuse the blood back to the descending aorta 10 in vivo, achieving outside aortic blood counterpulsation.

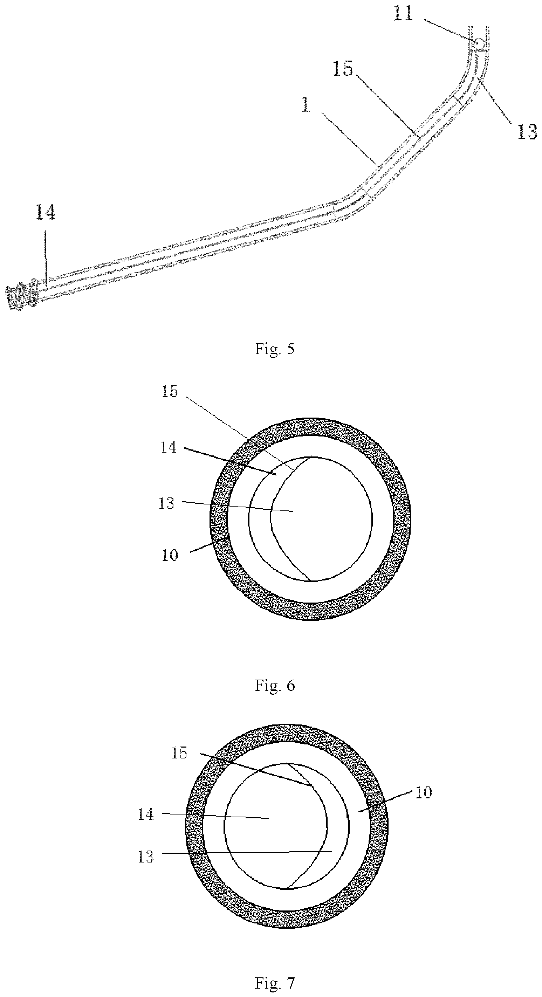

In a more preferred embodiment, as shown in Fig. 5, a second diaphragm 15 for dividing an interior of the catheter into a blood drawing cavity 13 and a perfusion cavity 14 is further disposed inside the in vivo catheter 1, one end of the second diaphragm 15 is located at a beginning of the perforated section of the in vivo catheter 1, and the other end of the second diaphragm 15 is located at a tip of the in vitro end of the in vivo catheter 1.

Specifically, both sides of the second diaphragm 15 are adhered to an inner side wall of the in vivo catheter 1, and both ends of the second diaphragm 15 are inclined such that an inner end of the blood drawing cavity 13 and an outer end of the perfusion cavity 14 are each of a flared structure, as shown in Fig. 5, for providing guidance for the flow of blood in the in vivo catheter, to ensure the smooth access of the blood.

More preferably, the second diaphragm 15 is made of an elastic thin film. The thin film is made of an elastic medical polymer material such as silicone rubber, polyurethane, PTFE, PU, Pebax or PE. The optimal hardness range of the elastic material is less than 30. During blood drawing, the elastic thin film deforms to the left, i.e. extruding the perfusion cavity 14, such that the blood drawing cavity 13 increases and the perfusion cavity 14 decreases, as shown in Fig. 6. During perfusion, the thin film deforms to the right, i.e., extruding the blood drawing cavity 13, such that the perfusion cavity 14 increases and the blood drawing cavity 13 decreases, as shown in Fig. 7. As a result, the adaptive adjustment is achieved during drawing and perfusion. In an initial state, the thin film is centralized to equally divide the lumen of the catheter. That is, the blood drawing cavity 13 and the perfusion cavity 14 have the same cross section area. The reason for designing the dual-cavity structure lies in that: in case of a single-channel catheter, the blood in the lumen always repeats the process of acceleration-deceleration-stop-reverse acceleration→reverse→deceleration→stop during blood drawing and perfusion, and in this process, the liquid cannot be fed back quickly due to high frequency and fast speed, naturally leading to vacuum and thus conditions such as mutual collision and serious turbulence; and in case of dual channels provided, the blood may be drawn from one end and perfused back from the other end, without mutual influence, which may increase the corresponding speed and eliminate vacuum and turbulence and consequently greatly reduce the required external power. Therefore, the catheter of the dual-cavity structure of the present application can ensure the unidirectional flow of the blood. Furthermore, in case of one inelastic thin film provided to divide a lumen into two cavities, the cross section of a single cavity becomes very small and is less than or equal to half of the original cross section. In this case, the difficulty in drawing and perfusion increases. Therefore, the creatively disposed elastic thin film with both ends provided with flared structures according to the present application can effectively guarantee the unidirectional flow of blood and allow for maximum unidirectional cross section of the blood in respective process, thereby greatly reducing the external power for better implementation of the unidirectional flow of blood.

More specifically, the in vivo catheter 1 and the in vitro catheter 3 are each made of a polymer material such as PTFE, PU, Pebax or PE and prepared by a blow molding or extrusion process, and the second diaphragm 15 is made of silicone rubber, polyurethane, PTFE, PU, Pebax, PE, or other elastic medical polymer materials having a hardness of less than 30.

In this embodiment, the controller 4 is an existing IABP system controller for supplying a gas to the inflation cavity 24. Preferably, a helium pump is disposed in the controller 4 to supply helium to the inflation cavity 24.

The controller 4 has the following functions: 1) waveform display: ECG, Ap and BP waveforms, ECG adjustable piston action interval, and accurate display of catheter pressure; 2) physiological data display: heart rate, assisted systolic/diastolic/mean/counterpulsation blood pressure, unassisted systolic/diastolic/mean blood pressure; 3) icon display: battery capacity, piston status, and accurate display of the pressure value in the respiratory cavity; 4) control mode: single touch screen control, button control, alarm angle control, and dual control of key/common functions: touch screen/buttons for auxiliary start, auxiliary frequency, screen freeze, print, and guide line setting; 5) operating mode: including automatic and manual modes, which can be switched without affecting the normal counterpulsation and with the original settings reserved automatically, where the automatic mode is available for automatic selection of signal source, trigger mode (6 types), time phase algorithm and optimal ECG lead (7 types), and real-time evaluation of ECG guide status, and the manual mode is available for selection of signal source, trigger mode, and ECG lead, and adjustment of time phase; 6) trigger mode: 7 types, including pattern mode, peak mode, Aifb mode, pacemaker V/A-V mode, pacemaker A mode, AP mode, and inboard setting mode; 7) balloon delay analysis: real-time calculation of balloon perfusion speed and evaluation of R-wave deflation safety; 8) auxiliary frequency: 4 types, including 1:1/1:2/1:4/1:8; 9) counterpulsation frequency: up to 200 times/min; 10) counterpulsation capacity: 0-150 ml each time, with the adjustable accuracy of 0.5 ml; 11) automatic water removal: automatic removal of condensed water (produced during cooling of a driver that produces heat during working, and needing removal before the device is used) every 20 times, without affecting normal assistance; 12) patient data report: display and print of all the recorded patient information related to counterpulsation; 13) power-on self-test checklist: indicating function self-test results in a list; and 14) alarm history: display and print of the most recent 100 alarms.

Of course, the controller 4 may also have other structures, as long as similar functions of the IABP system controller can be achieved. The gas supplied by the controller 4 may also be air, nitrogen or other safe gases, and the controller may also supply normal saline, water for injection, purified water or other safe liquids, as long as the design of the controller 4 is made easier and more stable.

According to the outside aortic counterpulsation device with the diaphragm cavity of the present invention, by means of the diaphragm cavity, the inflation cavity is disposed in vitro instead of in vivo as the IABP, since the length and diameter of the aortic blood vessel of a human body are limited in vivo, and the IABP device is available at most in three capacities, 30 cc, 40 cc, and 50 cc, and can thus supply the maximal cardiac output of 1 L/min at most; by placing the inflation cavity in vitro, its size, shape, and volume can be designed according to the maximum cardiac output to allow, for example, the volume of the inflation cavity to reach more than 150 ml and the actual cardiac output to be greater than 9 L/min, which thoroughly overcomes the huge defect of insufficient cardiac output of the IABP; and furthermore, the overall device still retains all the advantages of the IABP.

The above description only provides preferred embodiments of the present invention, and is not intended to limit the present invention in any form. Some simple alternations, equivalent variations or modifications made by those skilled in the art by using the technical content disclosed above shall fall within the protection scope of the present invention.

## Claims

1. An outside aortic counterpulsation device with a diaphragm cavity, comprising an in vivo catheter, the diaphragm cavity, an in vitro catheter and a controller that are connected in sequence,
wherein the in vivo catheter is used for extending into a descending aorta, and a side wall of an extending end of the in vivo catheter is provided with a plurality of through holes;
the diaphragm cavity comprises a cavity body and a first diaphragm disposed inside the cavity body, the first diaphragm is an elastic diaphragm body for dividing the cavity body into a blood cavity and an inflation cavity, the blood cavity is in airtight connection with an in vitro end of the in vivo catheter by means of a first fitting of the cavity body, the inflation cavity is in airtight connection with an end of the in vitro catheter by means of a second fitting of the cavity body, and the other end of the in vitro catheter is connected to the controller; and
the controller supplies an inflation or deflation gas to the inflation cavity, during inflation of the inflation cavity, the first diaphragm bulges towards the blood cavity, a space of the blood cavity is occupied to push blood in the blood cavity back into a body, and during deflation of the inflation cavity, the first diaphragm retracts, and the space of the blood cavity increases to draw blood from the descending aorta into the blood cavity, achieving an outside aortic blood counterpulsation effect.

2. The outside aortic counterpulsation device with the diaphragm cavity according to claim 1, wherein the first diaphragm is made of silicone rubber, polyurethane, PTFE or other elastic materials having a hardness of less than 30, with a thickness of 0.3-2 mm, and a gas volume of the inflation cavity is more than 150 ml after inflation.

3. The outside aortic counterpulsation device with the diaphragm cavity according to claim 2, wherein the cavity body is made of a rigid PEEK, PPSU, PU, PC or PE material, or a silicone rubber material having a hardness of more than 65.

4. The outside aortic counterpulsation device with the diaphragm cavity according to claim 1, wherein the first and second fittings of the cavity body are each provided with a Luer fitting.

5. The outside aortic counterpulsation device with the diaphragm cavity according to claim 1, wherein a pressure sensor is disposed on an outer side of a tip of an inner end of the in vivo catheter.

6. The outside aortic counterpulsation device with the diaphragm cavity according to claim 1, wherein the in vivo catheter has a lumen diameter of 2.2-9 mm, a perforated section of the in vivo catheter has a length of 320-520 mm, each of the through holes has a diameter of 1-4 mm, and a spacing between every two adjacent through holes is 1-10 mm.

7. The outside aortic counterpulsation device with the diaphragm cavity according to any one of claims 1 to 6, wherein a second diaphragm for dividing an interior of the in vivo catheter into a blood drawing cavity and a perfusion cavity is further disposed inside the in vivo catheter, one end of the second diaphragm is located at a beginning of the perforated section of the in vivo catheter, and the other end of the second diaphragm is located at a tip of the in vitro end of the in vivo catheter.

8. The outside aortic counterpulsation device with the diaphragm cavity according to claim 7, wherein both sides of the second diaphragm are adhered to an inner side wall of the in vivo catheter respectively, and both ends of the second diaphragm are inclined such that an inner end of the blood drawing cavity and an outer end of the perfusion cavity are each of a flared structure.

9. The outside aortic counterpulsation device with the diaphragm cavity according to claim 8, wherein the second diaphragm is made of an elastic thin film.

10. The outside aortic counterpulsation device with the diaphragm cavity according to claim 9, wherein the in vivo catheter and the in vitro catheter are each made of a polymer material such as PTFE, PU, Pebax or PE and prepared by a blow molding or extrusion process, and the second diaphragm is made of silicone rubber, polyurethane, PTFE, PU, Pebax, PE, or other elastic medical polymer materials having a hardness of less than 30.
